# FASCICULE DE BREVET EUROPEEN

(11) **EP 0 952 109 B1**
(45) Date de publication et mention de la délivrance du brevet: **10.10.2001**
(21) Numéro de dépôt: 99111127.9
(22) Date de dépôt: 16.11.1990
(51) Int. Cl.: C01B 21/16, B01D 3/06, F28F 21/08

(54) **Procédé de vaporisation d'une solution d'hydrate d'hydrazine**
Verfahren zur Verdampfung einer Hydrazinhydratlösung
Process for the evaporation of a hydrazine hydrate solution

(30) Priorité: 04.12.1989 FR 8915968
(43) Date de publication de la demande: 27.10.1999
(62) Demande divisionnaire de: 90403246.3
(73) Titulaire: Atofina, 92800 Puteaux (FR)
(72) Inventeur: Krempf, Gérard, 69003 Lyon (FR); Collier, Bertrand, 62250 La Barthe de Neste (FR); Tellier, Pierre, 69110 Sainte-Foy-Les-Lyon (FR); Pleuvry, Jean-Pierre, 62250 La Barthe de Neste (FR)

(56) Documents cités:
- FR-A- 1 548 921
- FR-A- 2 323 635
- J.R.PUDLOCK: "Corrosion-resistant shell and tube heat-exchangers costs compared." OIL AND GAS JOURNAL, vol. 75, no. 37, septembre 1977 (1977-09), pages 101-102, XP002115689 Tulsa (USA)
- Z.H. AYUB: "Tubeside erosion/corrosion in heat exchangers." HEATING, PIPING AND AIR CONDITIONING, vol. 59, no. 12, décembre 1987 (1987-12), pages 81-82, XP002115690 Stanford (USA)

## Description

La présente invention concerne un procédé de vaporisation d'une solution d'hydrate d'hydrazine.

La production industrielle de l'hydrate d'hydrazine se fait selon les procédés RASCHIG, BAYER et ATOCHEM.

Dans le procédé RASCHIG on oxyde l'ammoniac par un hypochlorite pour obtenir une solution diluée d'hydrate d'hydrazine qu'il faut ensuite concentrer par distillation.

Le procédé BAYER est une variante du procédé RASCHIG qui consiste à déplacer un équilibre chimique en piégeant, à l'aide d'acétone, l'hydrazine formée sous forme d'azine (CH₃)₂C=N-N=C(CH₃)₂. L'azine est ensuite isolée puis hydrolysée en hydrate d'hydrazine.

Le procédé ATOCHEM consiste à oxyder un mélange d'ammoniac et d'une cétone par l'eau oxygénée en présence d'un catalyseur pour faire directement l'azine qu'il suffit ensuite d'hydrolyser en hydrate d'hydrazine. Le procédé ATOCHEM est décrit dans de nombreux brevets, par exemple US 3 972 878, US 3 972 876, US 3 948 902 et US 4 093 656.

L'hydrolyse d'une azine en hydrate d'hydrazine est décrite dans les brevets US 4 724 133 SCHIRMANN et al, US 4 725 421 SCHIRMANN et al et GB 1 164 460. Cette hydrolyse s'effectue dans une colonne de distillation qu'on alimente avec de l'eau et de l'azine, en tête on récupère la cétone et en pied l'hydrate d'hydrazine. Dans le fond d'une colonne d'hydrolyse comme dans le fond d'une colonne de concentration d'hydrate d'hydrazine, il existe un dispositif pour vaporiser l'hydrate d'hydrazine.

La présente invention concerne l'utilisation de matériaux particuliers pour réaliser la surface d'un dispositif de vaporisation d'une solution d'hydrate d'hydrazine.

La demanderesse a découvert que la décomposition de l'hydrazine varie selon le matériau qui constitue la surface de transfert de l'énergie de vaporisation et que cette propriété était valable aussi bien (i) pour une vaporisation consistant à chauffer la solution d'hydrate d'hydrazine essentiellement en phase liquide puis à détendre cette solution que (ii) pour une vaporisation conventionnelle dans un bouilleur à thermosiphon, un serpentin noyé et en général tout dispositif dans lequel la vaporisation se produit au contact de la surface de chauffe.

La demanderesse a ainsi découvert qu'on peut classer dans l'ordre le titane, les oxydes de chrome, l'aluminium, l'acier ordinaire, l'inox 304, l'inox 316, le nickel et ses alliages et le zirconium. Le titane provoquant moins de décomposition que le zirconium. L'inox 304 et 316 sont les désignations usuelles des aciers inoxydables selon la norme AISI (American Iron and steel Institute).

Il est clair que le matériau du dispositif de vaporisation d'une solution d'hydrazine concerne le matériau au contact de la solution d'hydrate d'hydrazine et non pas le matériau au contact du fluide de chauffage. Les oxydes de chrome sont utilisés de préférence sous forme de revêtement d'une surface métallique.

La présente invention fournit donc un procédé de vaporisation d'une solution d'hydrate d'hydrazine sous pression,
caractérisé en ce que le matériau qui constitue la surface de transfert de l'énergie de vaporisation est en titane ou en oxyde de chrome.

La demanderesse a aussi constaté que si on utilise un rebouilleur à thermosiphon, un serpentin noyé dans le fond de la colonne ou un faisceau tubulaire noyé dans le fond de la colonne et en règle générale un rebouilleur où la vaporisation se produit au contact de la surface de chauffe, on observe une décomposition de l'hydrate d'hydrazine. La demanderesse a constaté que si on vaporise l'hydrate d'hydrazine par détente et non pas au contact de la surface de transfert de l'énergie thermique, on peut réduire sensiblement la décomposition.

La présente invention s'applique donc à un mode de vaporisation de la solution d'hydrate d'hydrazine où la vaporisation se produit au contact de la surface de chauffe.

La présente invention est particulièrement utile pour vaporiser des solutions aqueuses d'hydrate d'hydrazine à des températures supérieures à 100°C, et de préférence comprises entre 130 et 220°C. L'invention est ainsi particulièrement utile pour le bouilleur en pied d'une colonne d'hydrolyse d'azine ou d'hydrazone en hydrazine.

La solution d'hydrate d'hydrazine peut être une solution d'eau et d'hydrazine en proportions comprises entre l'hydrazine (ou l'hydrate) très diluée dans l'eau et l'hydrate d'hydrazine. La solution d'hydrate d'hydrazine peut être une solution contenant aussi une azine, une hydrazone, des cétones ; cette solution pouvant être aqueuse ou anhydre.

On désigne respectivement par azine et hydrazone les produits de formule : 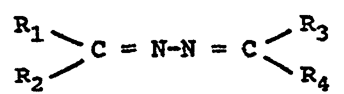 et 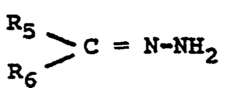 dans lesquels R₁ à R₆ sont identiques ou différents et désignent de l'hydrogène, un radical alkyl linéaire ayant de 1 à 12 atomes de carbone, un radical alkyl ramifié ou cycloalkyl ayant de 3 à 12 atomes de carbone, un radical aryl ayant de 6 à 12 atomes de carbone. Les radicaux R₁ à R₆ reliés au même atome de carbone de l'azine ou de l'hydrazone peuvent être eux-mêmes reliés et représenter ensemble un radical alkylène linéaire ou ramifié ayant de 3 à 12 atomes de carbone.

Tous les radicaux R₁ à R₆ précédents peuvent aussi être substitués par un chlore, un brome, un fluor ou un groupe nitro, hydroxy, alcoxy ou une fonction ester. L'invention est particulièrement utile pour l'acétone azine :

CH₃(CH₃)C=N-N=C(CH₃)CH₃ ;

la méthyléthylcétazine C₂H₅(CH₃)C=N-N=C(CH₃)C₂H₅, et les hydrazones correspondantes.

Pour chauffer cette solution essentiellement en phase liquide, il suffit de mettre la phase liquide en pression pendant qu'on la chauffe, c'est-à-dire que la solution d'hydrate d'hydrazine absorbe l'énergie thermique sous forme d'une augmentation de sa température puis on détend cette solution et l'énergie précédente est restituée sous forme d'une vaporisation.

On ne sortirait pas du cadre de l'invention si une partie de l'énergie thermique était consommée par une réaction d'hydrolyse d'une azine et/ou d'une hydrazone en hydrazine ou hydrazone correspondante.

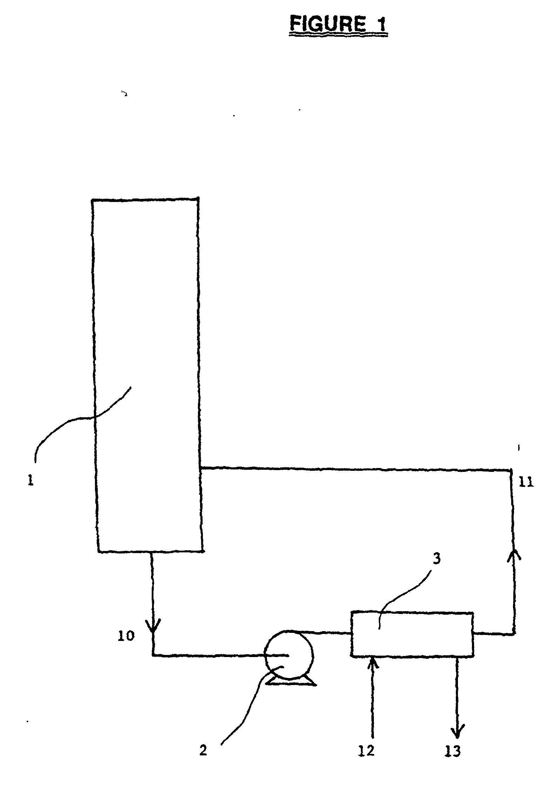

On ne sortirait pas du cadre de l'invention si pendant le chauffage de la solution d'hydrate d'hydrazine, une fraction était vaporisée, cette fraction pouvant représenter jusqu'à 3 ou 5 % en poids de la solution qui est chauffée puis détendue. La figure 1 décrit un mode de réalisation préféré de l'invention.

Dans cette figure 1, 1 représente une colonne à distiller, 2 une pompe et 3 un échangeur de chaleur. La solution d'hydrate d'hydrazine en pied de la colonne 1 est transportée via le tuyau 10 par la pompe 2 à travers l'échangeur 3 puis retourne dans la colonne par la tuyauterie 11. Par simplification on n'a pas représenté le soutirage de pied de la colonne 1 qui peut être par exemple au refoulement de 2. La pompe 2 permet de donner à la solution d'hydrate d'hydrazine une pression supérieure à celle du fond de la colonne 1. lui permettant de circuler dans l'échangeur 3 et le tuyau 11. Cette pression permet de maintenir la solution en phase essentiellement liquide dans l'échangeur 3. L'échangeur 3 est alimenté en 12 par de la vapeur ou un fluide caloporteur qui est évacué en 13. On peut disposer en un point quelconque du tuyau 11 un dispositif pour créer une perte de charge, tel qu'une vanne ou un diaphragme. L'homme de métier peut choisir facilement de mettre ou de ne pas mettre un dispositif de perte de charge sur le tuyau 11 en fonction des pertes de charge créés par l'échangeur 3 et la tuyauterie 11. La pression relative fournie par la pompe, c'est-à-dire la différence de pression entre le refoulement de la pompe et le pied de la colonne peut être quelconque mais elle est habituellement supérieure à 0,5 bar et de préférence comprise entre 1 et 10 bars.

On ne sortirait pas du cadre de l'invention en disposant l'échangeur 3 et la pompe 2 très en-dessous du niveau du fond de la colonne 1. On créé ainsi une pression supplémentaire sur la solution d'hydrate d'hydrazine dans l'échangeur 3. On pourrait aussi selon cette variante si la différence de niveau est suffisante pour maintenir dans l'échangeur 3 la solution en phase liquide, supprimer la pompe 2. Cette forme de réalisation n'a d'intérêt que si la colonne 1 est à une hauteur suffisante au-dessus du sol.

La colonne 1 peut être aussi un simple réservoir dans lequel on effectue une réaction nécessitant une vaporisation et qui serait munie d'une alimentation et éventuellement d'un soutirage en tête avec ou sans reflux. Cette colonne 1 étant insérée éventuellement dans un procédé. C'est par exemple un réservoir dans lequel on effectue une réaction endothermique.

### EXEMPLE

Une capacité de volume 300 l contenant une solution d'hydrate d'hydrazine est munie d'un échangeur de chaleur extérieur de 5 m² disposé verticalement avec retour dans la partie supérieure de la capacité. On peut aussi disposer une pompe pouvant débiter 20 m³/heure avec une hauteur manométrique de 2 bars entre la capacité et l'échangeur et un disphragme en sortie pour le faire fonctionner en bouilleur à circulation forcée. On condense 250 Kg/h de vapeur à 18 bars dans l'échangeur. La capacité est munie d'un condenseur de reflux total pour éliminer l'énergie fournie à l'échangeur et d'un dégazage pour recueillir l'azote, l'hydrogène et l'ammoniac provenant de la décomposition de l'hydrazine et ainsi mesurer cette décomposition.

L'ensemble est construit en acier et on a essayé des échangeurs en différents matériaux.

Pour tester chaque matériau, on remplit la capacité avec 50 kg d'hydrate d'hydrazine (N₂H₄, H₂O) dilué dans 250 kg d'eau, puis on envoie de la vapeur 18 bars pour faire fonctionner l'échangeur en bouilleur (on condense 250 kg/heure). On mesure au bout d'une heure la quantité d'hydrazine restant dans la capacité. Cette valeur est corellée par la mesure du dégazage. La pression dans la capacité est de 9 bars relatifs et la température 180°C.

### a) On fonctionne en thermosiphon

Si on utilise un échangeur en inox 316, il reste au bout d'une heure 45 kg d'hydrate d'hydrazine, pour l'acier il en reste 47 kg et pour le titane 48,5 kg.

### b) On fonctionne avec la pompe

En utilisant un échangeur en inox 316 il reste au bout d'une heure 47,5 kg d'hydrate d'hydrazine.

## Revendications

1. Procédé de vaporisation d'une solution d'hydrate d'hydrazine sous pression, **caractérisé en ce que** le matériau qui constitue la surface de transfert de l'énergie de vaporisation est en titane ou en oxyde de chrome.

2. Procédé selon la revendication 1 **caractérisé en ce que** la température de la solution d'hydrate d'hydrazine est comprse entre 130 et 220°C.

3. Procédé selon la revendication 1 ou 2 **caractérisé en ce que** l'on utilise pour le bouilleur en pied d'une colonne d'hydrolyse d'azine ou d'hydrazone en hydrazine.

## Patentansprüche

1. Verfahren zur Verdampfung einer Hydrazinhydrat-Lösung unter Druck, **dadurch gekennzeichnet, daß** das Material, aus dem die Oberfläche besteht, die zur Übertragung der Energie für die Verdampfung dient, aus Titan oder Chromoxid besteht.

2. Verfahren nach Anspruch 1, **dadurch gekennzeichnet, daß** die Temperatur der Hydrazinhydrat-Lösung im Bereich von 130 bis 220 °C befindet

3. Verfahren nach Anspruch 1 oder 2, **dadurch gekennzeichnet, daß** es für den Siedekessel am Fuß einer Kolonne verwendet wird, in der ein Azin oder ein Hydrazon zum Hydrazin hydrolysiert wird.

## Claims

1. Process for the evaporation of a hydrazine hydrate solution under pressure, **characterized in that** the material which constitutes the surface for transfer of the energy of evaporation is made of titanium or is made of chromium oxide.

2. Process according to Claim 1, **characterized in that** the temperature of the hydrazine hydrate solution is between 130 and 220°C.

3. Process according to Claim 1 or 2, **characterized in that** it is used for the bottom boiler of a column for the hydrolysis of azine or hydrazone to hydrazine.
